# EUROPEAN PATENT APPLICATION

(11) **EP 4 478 265 A1**
(43) Date of publication of application: **18.12.2024**
(21) Application number: 24177012.2
(22) Date of filing: 21.05.2024
(51) Int. Cl.: G06N 20/00, G06N 5/045, G16H 10/00

(54) **APPARATUS, METHOD, AND COMPUTER PROGRAM PRODUCT FOR MULTI-LABEL CLASSIFICATION USING ADAPTED MULTI-CLASS CLASSIFICATION MODEL**

(30) Priority: 12.06.2023 US 202318333198
(71) Applicant: Honeywell International Inc., Charlotte, NC 28202 (US)
(72) Inventor: SUBBER, Waad, Charlotte, 28202 (US); SINGH, Ankit, Charlotte, 28202 (US)
(74) Representative: Haseltine Lake Kempner LLP

(57) **Abstract**

A multi-label classification system performs multi-label classification using an adapted multi-class classification model in conjunction with pre-classification and post-classification processing and data transformation. An original, multi-label training data set in which objects are classified into at least one but possibly multiple original classification sets is transformed into an adapted, multi-class training data set in which the same objects are each classified into a combined classification set representing a combination of all of the individual, original classification sets into which the object is classified in the original training data set. The adapted training data set is used to train a multi-class classification model, which trained model is used to generate a multi-class classification of objects with respect to an input data set. The multi-class classification is transformed into a multi-label classification.

## Description

### TECHNICAL FIELD

Embodiments of the present disclosure generally relate to machine learning classification models, and specifically, in some examples, to machine learning models for multi-label classification of quality management events.

### BACKGROUND

Applicant has identified example technical challenges and difficulties associated with machine learning models for multi-label classification. Through applied effort, ingenuity, and innovation, Applicant has solved problems relating to machine learning models for multi-label classification.

### BRIEF SUMMARY

According to one aspect, example embodiments of the present disclosure include an apparatus comprising at least one processor and at least one non-transitory memory comprising program code stored thereon. The at least one non-transitory memory and the program code are configured to, with the at least one processor, cause the apparatus to at least: transform an original training data set corresponding to a multi-label classification task into an adapted training data set corresponding to a multi-class classification task based at least in part on a predefined transformation protocol, wherein a multi-class classification model configured to perform the multi-class classification task is trained based at least in part on the adapted training data set; transform multi-class output data generated with respect to an input data set using the trained multi-class classification model into multi-label output data corresponding to the multi-label classification task based at least in part on the predefined transformation protocol; and cause performance of at least one enterprise management operation based at least in part on the multi-label output data.

In some embodiments, the original training data set comprises a multi-label classification of training objects each into at least one original classification set of a plurality of original classification sets, in which at least one training object of the training objects is classified into a plurality of different original classification sets. The adapted training data set may comprise a multi-class classification of the training objects each into a corresponding combined classification set of at least one combined classification set, in which the combined classification set corresponding to each particular training object of the training objects represents a combination of each original classification set into which the particular training object is classified in the multi-label classification of the original training data set. The multi-class output data may comprise a classification of production objects from the input data set each into a corresponding combined classification set of the at least one combined classification set from the adapted training data set. The multi-label output data may comprise a classification of the production objects from the input data set each into at least one original classification set of the plurality of original classification sets from the original training data set.

In some embodiments, the at least one non-transitory memory and the program code are configured to, with the at least one processor, further cause the apparatus to at least train the multi-class classification model based at least in part on the adapted training data set.

In some embodiments, the at least one non-transitory memory and the program code are configured to, with the at least one processor, further cause the apparatus to at least generate the multi-class output data with respect to the input data set using the trained multi-class classification model.

In some embodiments, transforming the original training data set into the adapted training data set comprises, for each particular training object of training objects classified in the original training data set, combining all original classification labels corresponding to original classification sets into which the particular training object is classified in the original training data set into a combined classification label comprising all of the original classification labels for the particular training object delimited via a label combiner operator defined according to the predefined transformation protocol.

In some embodiments, transforming the multi-class output data into the multi-label output data comprises, for each particular production object of production objects classified in the multi-class output data, parsing, based at least in part on a label combiner operator defined according to the predefined transformation protocol, a combined classification label corresponding to a combined classification set into which the particular production object is classified in the multi-class output data into at least one original classification label corresponding to an original classification set from the original training data set into which the particular production object is to be classified in the multi-label output data, wherein the combined classification label comprises the at least one original classification label delimited via the label combiner operator.

In some embodiments, the multi-label classification task includes classification of text objects containing text characterizing quality management events associated with products produced by an enterprise into classification sets corresponding to medical terms from a medical dictionary.

According to another aspect, embodiments of the present invention feature a method comprising: transforming an original training data set corresponding to a multi-label classification task into an adapted training data set corresponding to a multi-class classification task based at least in part on a predefined transformation protocol, wherein a multi-class classification model configured to perform the multi-class classification task is trained based at least in part on the adapted training data set; transforming multi-class output data generated with respect to an input data set using the trained multi-class classification model into multi-label output data corresponding to the multi-label classification task based at least in part on the predefined transformation protocol; and causing performance of at least one enterprise management operation based at least in part on the multi-label output data.

According to another aspect, embodiments of the present invention feature a computer program product comprising at least one non-transitory computer-readable storage medium having computer-readable program code portions stored therein. The computer-readable program code portions comprise an executable portion configured to: transform an original training data set corresponding to a multi-label classification task into an adapted training data set corresponding to a multi-class classification task based at least in part on a predefined transformation protocol, wherein a multi-class classification model configured to perform the multi-class classification task is trained based at least in part on the adapted training data set; transform multi-class output data generated with respect to an input data set using the trained multi-class classification model into multi-label output data corresponding to the multi-label classification task based at least in part on the predefined transformation protocol; and cause performance of at least one enterprise management operation based at least in part on the multi-label output data.

The above summary is provided merely for the purpose of summarizing some example embodiments to provide a basic understanding of some aspects of the present disclosure. Accordingly, it will be appreciated that the above-described embodiments are merely examples and should not be construed to narrow the scope or spirit of the present disclosure in any way. It will be appreciated that the scope of the present disclosure encompasses many potential embodiments in addition to those here summarized, some of which will be further described below. Other features, aspects, and advantages of the subject will become apparent from the description, the drawings, and the claims.

### BRIEF DESCRIPTION OF THE DRAWINGS

Having thus described the embodiments of the disclosure in general terms, reference now will be made to the accompanying drawings, which are not necessarily drawn to scale, and wherein:
FIG. 1 illustrates an exemplary block diagram of an environment in which embodiments of the present disclosure may operate;
FIG. 2 illustrates an exemplary block diagram of an example apparatus that may be specially configured in accordance with an example embodiment of the present disclosure;
FIG. 3 illustrates an exemplary multi-label classification system, in accordance with at least some example embodiments of the present disclosure;
FIG. 4 illustrates example data objects used by and produced by a multi-label classification system at various stages, in accordance with at least some example embodiments of the present disclosure; and
FIG. 5 is a flowchart depicting an example process for performing multi-label classification using an adapted multi-class classification model, in accordance with at least some example embodiments of the present disclosure.

### DETAILED DESCRIPTION

Some embodiments of the present disclosure now will be described more fully hereinafter with reference to the accompanying drawings, in which some, but not all, embodiments of the disclosure are shown. Indeed, embodiments of the disclosure may be embodied in many different forms and should not be construed as limited to the embodiments set forth herein, rather, these embodiments are provided so that this disclosure will satisfy applicable legal requirements. Like numbers refer to like elements throughout.

As used herein, terms such as "front," "rear," "top," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

The use of the term "circuitry" as used herein with respect to components of a system or an apparatus should be understood to include particular hardware configured to perform the functions associated with the particular circuitry as described herein. The term "circuitry" should be understood broadly to include hardware and, in some embodiments, software for configuring the hardware. For example, in some embodiments, "circuitry" may include processing circuitry, communication circuitry, input/output circuitry, and the like. In some embodiments, other elements may provide or supplement the functionality of particular circuitry. Alternatively or additionally, in some embodiments, other elements of a system and/or apparatus described herein may provide or supplement the functionality of another particular set of circuitry. For example, a processor may provide processing functionality to any of the sets of circuitry, a memory may provide storage functionality to any of the sets of circuitry, communications circuitry may provide network interface functionality to any of the sets of circuitry, and/or the like.

The term "electronically coupled," "electronically coupling," "electronically couple," "in communication with," "in electronic communication with," or "connected" in the present disclosure refers to two or more elements or components being connected through wired means and/or wireless means, such that signals, electrical voltage/current, data and/or information may be transmitted to and/or received from these elements or components.

Machine learning systems have been proposed to solve classification problems within various contexts and with respect to various subject matter domains. In these classification problems, in some examples, objects represented in data are classified into categories based on features of the data associated with the objects being classified. For the purpose of illustration, in some examples, text objects containing various discrete textual descriptions may be classified into categories based on features of the text objects such as the presence of certain words in the text, patterns in how certain words or types of words are arranged with respect to each other, semantic and/or numeric characteristics derived from the text, and/or various data and/or metadata attributes associated with the text objects, among other examples. A (e.g., annotated) training data set comprising sample text objects each associated with classification labels may be used (e.g., in conjunction with a machine learning algorithm) to develop, define, and/or train a classification model capable of classifying unlabeled and/or unclassified text objects of a given input data set into the various categories (or classification sets) reflected in the training data set.

Types of classification problems to which these machine learning systems may be directed include multi-label classification and multi-class classification, to name a few examples. In multi-label classification, a given object may be classified into multiple, non-exclusive classification sets, for example, with possibly multiple classification labels representing different classification sets being assigned to the object. On the other hand, in multi-class classification, a given object may be classified into exactly one classification set from among at least three classification sets.

In some exemplary, domain-specific scenarios, enterprises such as businesses or other organizations may produce products (e.g., manufactured goods, provided services) of various types via various research, development, manufacturing, distribution, and/or service provision processes associated with production of these products. In many such cases, these enterprises may develop, implement, and/or perform quality management systems, processes, and/or operations with respect to these research, development, manufacturing, distribution, and/or service provision processes with an aim toward ensuring that the quality of the products being produced meet a certain standard at a certain level of consistency. Sometimes, certain aspects of such quality management may even be required by laws and/or regulations, and some quality management systems, processes, and/or operations may be devised and/or configured to ensure that research, development, manufacturing, distribution, and/or service provision processes associated with production of these products are in compliance with applicable laws and/or regulations. Quality management may be especially important with respect to production of certain types of products in particular, including medical devices, pharmaceutical products, and/or medical or life sciences services. For example, these products may be subject to a relatively high degree of quality management regulation.

Quality management software systems (also referred to herein as quality management systems) may be used to implement and/or facilitate quality management measures undertaken by enterprises with respect to the production of their products and/or provision of their services. More particularly, in one example, it may be possible for a quality management system to facilitate the process of receiving, generating, and/or aggregating any data associated with production of products that is required for quality management purposes. A quality management system may be configured to log quality management events based on input from various sources, including possibly internal enterprise data associated with production of the relevant products, data generated and/or received in connection with provision or use of the relevant products, and/or data characterizing and/or derived from communications with various entities associated with production, consumption, and/or use of the relevant products. In one example, the system may be configured to enable users to generate and fill out various types of forms associated with quality management, including complaint forms, supplier quality management forms, audit forms, risk forms, and/or quality event forms, to list a few examples. This data may be collected, archived, and consulted in order to provide visibility into various quality management objectives and/or operations with respect to the various research, development, manufacturing, distribution, and/or service provision processes subject to quality management and/or to initiate and/or cause performance of various quality management operations with respect to the processes in question. In some examples, quality management systems may be configured to present status data and/or generate and present insights (e.g., predictions and/or recommendations) for optimizing development and performance of quality management operations. These insights are often generated using machine learning models, which may be developed, configured, and/or trained using one or more machine learning algorithms.

Some of the data that may be collected by quality management systems (e.g., quality management events) may comprise text, including, for example, text characterizing how products were provided, consumed, and/or used, text characterizing particular contexts in which products were provided, consumed, and/or used, and/or text characterizing certain effects resulting from provision, consumption, and/or use of the products, among other examples. Text objects (e.g., comprised by quality management events) may contain text that represents and/or describes customer complaints arising from provision, consumption, and/or use of the products subject to quality management by the quality management system. In certain domains or contexts, it may be valuable or useful to perform classification tasks with respect to these text objects. In one example, it may be beneficial for a quality management system to classify each of these text objects into one or more classification sets representing terms defined in the Medical Dictionary for Regulatory Activities (MedDRA), which is a dictionary defining standard terms for various symptoms, signs, diseases, syndromes, and diagnoses, to list a few examples. In this case, it may be possible for a quality management system to be configured to perform a multi-label classification (e.g., using machine learning models and/or algorithms) with respect to the text objects and the MedDRA terms, assigning to each text object at least one (but possibly multiple) classification labels each corresponding to one of the MedDRA terms based on features of the text object. For the purpose of illustration, in one example, text from a quality management event describing coughing may be assigned classification labels representing multiple MedDRA terms associated with coughing, such as "flu," "cancer," and any other terms with a possible relevance to coughing. Thus, for example, quality management data may be enhanced as a result of this multi-label classification, as any MedDRA terms that may be pertinent to a particular text object can be automatically determined and associated with the text (or quality management event containing the text), which associations may aid in further analysis and/or interpretation of the data and/or performance of quality management operations in connection with the data.

However, existing approaches to multi-label classification present a number of challenges. For example, in some instances, it has been proposed to implement multi-label classification using a number of specialized binary classification models each independently trained with respect to an individual classification label, requiring each of these binary classification models to be used (via a corresponding specialized algorithm) for every given object to be classified. Scalability of this approach is limited, and performing a multi-label classification with respect to a sufficiently large number of classification labels may be time-consuming or may consume excessive processing and memory resources. These drawbacks may render multi-label classification impractical or even impossible in some contexts.

In various embodiments, the presently disclosed multi-label classification system is configured to perform a multi-label classification task using an adapted multi-class classification model in conjunction with pre-classification and post-classification processing and data transformation steps. More particularly, in one example, an original training data set corresponding to a multi-label classification task is transformed into an adapted training data set in which any individual classification labels assigned to a particular object are combined into a single, combined label comprising all of the individual classification labels delimited via a combiner operator. A multi-class classification model is then trained using the adapted training data set, and the trained model is used to generate multi-class predictions with respect to the combined labels reflected in the adapted training data. The combined labels indicated in these multi-class predictions are then parsed and transformed into multi-label predictions (e.g., based on the combiner operator, using a mapping of the combined labels to their corresponding individual labels). Accordingly, the multi-label classification system involves only a single (multi-class) classification task rather than multiple binary classification tasks, thus improving performance, reducing implementation complexity and resource consumption, expanding the range of applications of the pertinent multi-label classification functionality, and eliminating the need for multiple specialized algorithms and binary classification models as well as the need for non-standard accuracy and cost metrics for measuring the performance and for training the model, among other examples.

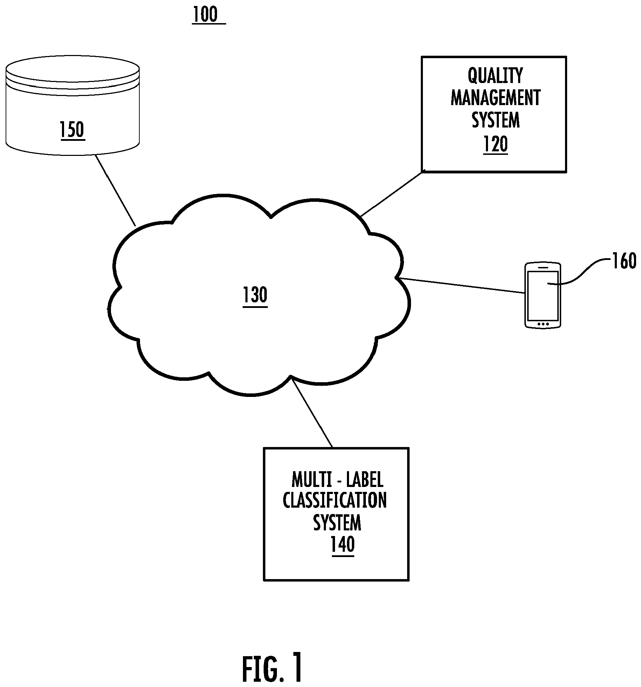

FIG. 1 illustrates an exemplary block diagram of an environment 100 in which embodiments of the present disclosure may operate. Specifically, FIG. 1 illustrates a multi-label classification system 140, a quality management system 120, one or more data repositories 150, and one or more user devices 160, all connected to a network 130.

The network 130 may be embodied in any of a myriad of network configurations. In some embodiments, the network 130 may be a public network (e.g., the Internet). In some embodiments, the network 130 may be a private network (e.g., an internal localized, or closed-off network between particular devices). In some other embodiments, the network 130 may be a hybrid network (e.g., a network enabling internal communications between particular connected devices and external communications with other devices). In various embodiments, the network 130 may include one or more base station(s), relay(s), router(s), switch(es), cell tower(s), communications cable(s), routing station(s), and/or the like. In various embodiments, components of the environment 100 may be communicatively coupled to transmit data to and/or receive data from one another over the network 130. Such configuration(s) include, without limitation, a wired or wireless Personal Area Network (PAN), Local Area Network (LAN), Metropolitan Area Network (MAN), Wide Area Network (WAN), and/or the like.

The one or more data repositories 150 may be configured to receive, store, and/or transmit data. In some embodiments, the one or more data repositories 150 may be configured to store enterprise data associated with production of one or more products by one or more enterprises (e.g., quality management data, product data, process data, system data, any data that may be used in connection with quality management operations including any data that may be included in and/or used to generate a training data set for a multi-label classification model, and/or any related object data and/or metadata). In some embodiments, the one or more data repositories 150 may be configured to store data associated with development, configuration, training, and/or use (e.g., by the multi-label classification system 140) of one or more machine learning models corresponding to multi-label and/or multi-class prediction tasks associated with quality management for production of the product(s), including, for example, original and/or adapted training data, transformation protocols for transforming training data and/or output data, any data defining trained model weights, parameters, and/or hyperparameters for trained classification models, multi-class and/or multi-label output data produced via trained classification models. In various embodiments, some or all of the data stored in the one or more data repositories 150 may be stored in a shared memory system shared between the quality management system 120 and/or the multi-label classification system 140 and/or may be otherwise accessible to the quality management system 120 and the multi-label classification system 140.

The one or more user devices 160 may be associated with and/or operated by users of the quality management system 120, the multi-label classification system 140, and/or one or more enterprise systems associated with the one or more products and/or production of the one or more products. In one example, the enterprise system(s) associated with the one or more products and/or production thereof may cause data associated with the production of the one or more products to be transmitted to, to be displayed on, and/or to be used to generate data or interface elements displayed on the user devices(s) 160 and/or may cause one or more interfaces (e.g., user interfaces) to be presented via the user device(s) 160, which interface(s) may be configured to receive input comprising and/or used to generate any data associated with the one or more products and/or production thereof, including, in some examples, product data, process data, and/or system data. In another example, the quality management system 120 may cause data associated with development, configuration, monitoring, tracking, optimization, and/or performance of quality management operations and/or objectives with respect to production of the product(s) to be transmitted to, to be displayed on, and/or to be used to generate data or interface elements displayed on the user devices(s) 160 and/or may cause one or more interfaces (e.g., user interfaces) to be presented via the user device(s) 160, which interface(s) may be configured to receive input comprising and/or used to generate any data associated with the quality management operations and/or objectives. In another example, the multi-label classification system 140 may cause data associated with development, configuration, training, and/or use of one or more machine learning models corresponding to multi-label and/or multi-class prediction tasks associated with quality management for production of the product(s) to be transmitted to, to be displayed on, and/or to be used to generate data or interface elements displayed on the user devices(s) 160 and/or may cause one or more interfaces (e.g., user interfaces) to be presented via the user device(s) 160, which interface(s) may be configured to receive input comprising and/or used to generate any data associated with the machine learning model(s).

The multi-label classification system 140 may be a computing system or device (e.g., server system) configured via hardware, software, firmware, and/or a combination thereof, to perform multi-label classification functionality comprising, for example, transforming original training data into adapted training data, training classification models, generating output data using the trained models, transforming multi-class output data into multi-label output data, and/or causing performance of enterprise management operations (e.g., quality management operations) based on the output data.

The quality management system 120 may be a computing system or device (e.g., server system) configured via hardware, software, firmware, and/or a combination thereof, to perform quality management operations with respect to production of the product(s), including, for example, presenting interface(s) configured to receive input used in performance of the quality management operations, receiving (e.g., via the presented interface(s)) the input used in performance of the quality management operations, generating, receiving, accessing, retrieving, and/or aggregating quality management events and/or any event data comprised by or derived therefrom, and/or performing the quality management operations based on the retrieved event data and/or quality management events. In some embodiments, the quality management system 120 may correspond to and/or comprise a quality management software system and/or quality management system similar to and/or performing similar functionality as a quality management software system and/or quality management system as defined and described above.

In various embodiments, the quality management system 120 and the multi-label classification system 140 may have varying degrees of integration with respect to each other. For example, the quality management system 120 and the multi-label classification system 140 may be configured to transmit and/or receive communications and/or data with respect to each other and/or access commonly accessible or shared data stores to exchange training data, input data, and/or output data associated with the classification models. In another example, the multi-label classification system 140 may be a sub-system of and/or otherwise integrated as part of the quality management system 120. In another example, the quality management system 120 and the multi-label classification system 140 may be sub-systems of and/or otherwise integrated as part of a greater enterprise management system or enterprise performance management system configured to perform one or more enterprise management operations (e.g., including quality management operations) with respect to one or more products produced by one or more enterprises and/or one or more processes and/or systems associated with production of the one or more products.

While FIG. 1 illustrates certain components as separate, standalone entities communicating over the network 130, various embodiments are not limited to this configuration. In other embodiments, one or more components may be directly connected and/or share hardware or the like.

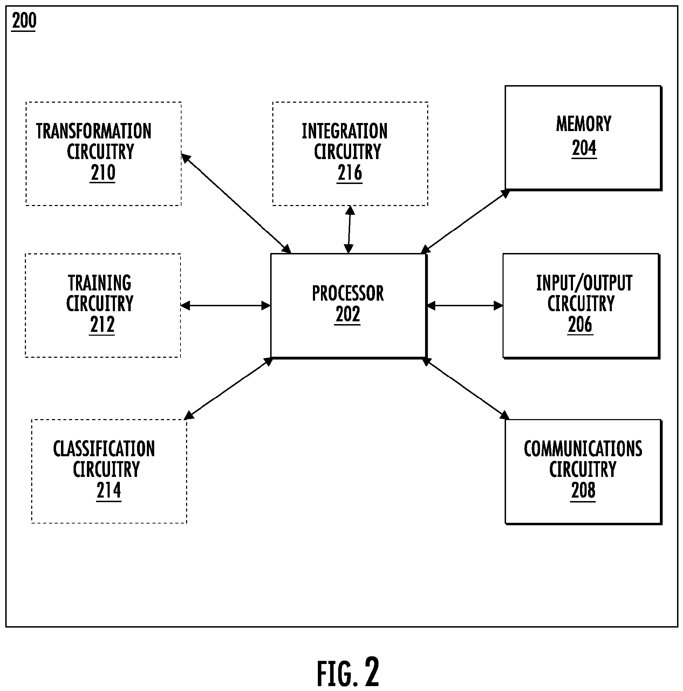

FIG. 2 illustrates an exemplary block diagram of an example apparatus that may be specially configured in accordance with an example embodiment of the present disclosure. Specifically, FIG. 2 depicts an example computing apparatus 200 ("apparatus 200") specially configured in accordance with at least some example embodiments of the present disclosure. Examples of an apparatus 200 may include, but is not limited to, one or more components of one or more operational systems associated with the one or more products and/or production of the one or more products, a multi-label classification system 140, a quality management system 120, data repositories 150, and/or user devices 160. The apparatus 200 includes processor 202, memory 204, input/output circuitry 206, communications circuitry 208, and/or transformation circuitry 210, training circuitry 212, classification circuitry 214, and/or integration circuitry 216. In some embodiments, the apparatus 200 is configured to execute and perform the operations described herein.

Although components are described with respect to functional limitations, it should be understood that the particular implementations necessarily include the use of particular computing hardware. It should also be understood that in some embodiments certain of the components described herein include similar or common hardware. For example, in some embodiments two sets of circuitry both leverage use of the same processor(s), memory(ies), circuitry(ies), and/or the like to perform their associated functions such that duplicate hardware is not required for each set of circuitry.

In various embodiments, a device, system, or apparatus, such as apparatus 200 of one or more components of one or more operational systems associated with the one or more products and/or production of the one or more products, a multi-label classification system 140, a quality management system 120, data repositories 150, and/or user devices 160, may refer to, for example, one or more computers, computing entities, desktop computers, mobile phones, tablets, phablets, notebooks, laptops, distributed systems, servers, or the like, and/or any combination of devices or entities adapted to perform the functions, operations, and/or processes described herein. Such functions, operations, and/or processes may include, for example, transmitting, receiving, operating on, processing, displaying, storing, determining, creating/generating, monitoring, evaluating, comparing, and/or similar terms used herein. In one embodiment, these functions, operations, and/or processes can be performed on data, content, information, and/or similar terms used herein. In this regard, the apparatus 200 embodies a particular, specially configured computing entity transformed to enable the specific operations described herein and provide the specific advantages associated therewith, as described herein.

Processor 202 or processor circuity 202 may be embodied in a number of different ways. In various embodiments, the use of the terms "processor" should be understood to include a single core processor, a multi-core processor, multiple processors internal to the apparatus 200, and/or one or more remote or "cloud" processor(s) external to the apparatus 200. In some example embodiments, processor 202 may include one or more processing devices configured to perform independently. Alternatively, or additionally, processor 202 may include one or more processor(s) configured in tandem via a bus to enable independent execution of operations, instructions, pipelining, and/or multithreading.

In an example embodiment, the processor 202 may be configured to execute instructions stored in the memory 204 or otherwise accessible to the processor. Alternatively, or additionally, the processor 202 may be configured to execute hard-coded functionality. As such, whether configured by hardware or software methods, or by a combination thereof, processor 202 may represent an entity (e.g., physically embodied in circuitry) capable of performing operations according to embodiments of the present disclosure while configured accordingly. Alternatively, or additionally, processor 202 may be embodied as an executor of software instructions, and the instructions may specifically configure the processor 202 to perform the various algorithms embodied in one or more operations described herein when such instructions are executed. In some embodiments, the processor 202 includes hardware, software, firmware, and/or a combination thereof that performs one or more operations described herein.

In some embodiments, the processor 202 (and/or co-processor or any other processing circuitry assisting or otherwise associated with the processor) is/are in communication with the memory 204 via a bus for passing information among components of the apparatus 200.

Memory 204 or memory circuitry embodying the memory 204 may be non-transitory and may include, for example, one or more volatile and/or non-volatile memories. In some embodiments, the memory 204 includes or embodies an electronic storage device (e.g., a computer readable storage medium). In some embodiments, the memory 204 is configured to store information, data, content, applications, instructions, or the like, for enabling an apparatus 200 to carry out various operations and/or functions in accordance with example embodiments of the present disclosure.

Input/output circuitry 206 may be included in the apparatus 200. In some embodiments, input/output circuitry 206 may provide output to the user and/or receive input from a user. The input/output circuitry 206 may be in communication with the processor 202 to provide such functionality. The input/output circuitry 206 may comprise one or more user interface(s). In some embodiments, a user interface may include a display that comprises the interface(s) rendered as a web user interface, an application user interface, a user device, a backend system, or the like. In some embodiments, the input/output circuitry 206 also includes a keyboard, a mouse, a joystick, a touch screen, touch areas, soft keys a microphone, a speaker, or other input/output mechanisms. The processor 202 and/or input/output circuitry 206 comprising the processor may be configured to control one or more operations and/or functions of one or more user interface elements through computer program instructions (e.g., software and/or firmware) stored on a memory accessible to the processor (e.g., memory 204, and/or the like). In some embodiments, the input/output circuitry 206 includes or utilizes a user-facing application to provide input/output functionality to a computing device and/or other display associated with a user.

Communications circuitry 208 may be included in the apparatus 200. The communications circuitry 208 may include any means such as a device or circuitry embodied in either hardware or a combination of hardware and software that is configured to receive and/or transmit data from/to a network and/or any other device, circuitry, or module in communication with the apparatus 200. In some embodiments the communications circuitry 208 includes, for example, a network interface for enabling communications with a wired or wireless communications network. Additionally or alternatively, the communications circuitry 208 may include one or more network interface card(s), antenna(s), bus(es), switch(es), router(s), modem(s), and supporting hardware, firmware, and/or software, or any other device suitable for enabling communications via one or more communications network(s). In some embodiments, the communications circuitry 208 may include circuitry for interacting with an antenna(s) and/or other hardware or software to cause transmission of signals via the antenna(s) and/or to handle receipt of signals received via the antenna(s). In some embodiments, the communications circuitry 208 enables transmission to and/or receipt of data from a user device and/or other external computing device(s) in communication with the apparatus 200.

Transformation circuitry 210 may be included in the apparatus 200 (e.g., an apparatus specifically corresponding to the multi-label classification system 140). The transformation circuitry 210 may include hardware, software, firmware, and/or a combination thereof, designed and/or configured to request, receive, process, generate, and transmit data, data structures, control signals, and electronic information for adapting multi-class classification models for performance of multi-label classification tasks, including performing any of the operations described herein with respect to generating adapted training data and/or generating multi-label output data.

Training circuitry 212 may be included in the apparatus 200 (e.g., an apparatus specifically corresponding to the multi-label classification system 140). The training circuitry 212 may include hardware, software, firmware, and/or a combination thereof, designed and/or configured to request, receive, process, generate, and transmit data, data structures, control signals, and electronic information for functionality related to training classification models, including performing any of the operations described herein with respect to training a multi-class classification model (e.g., using an adapted training data set).

Classification circuitry 214 may be included in the apparatus 200 (e.g., an apparatus specifically corresponding to the multi-label classification system 140). The classification circuitry 214 may include hardware, software, firmware, and/or a combination thereof, designed and/or configured to request, receive, process, generate, and transmit data, data structures, control signals, and electronic information for functionality related to performing classification tasks, including performing any of the operations described herein with respect to generating multi-class output data using a trained multi-class classification model.

Integration circuitry 216 may be included in the apparatus 200 (e.g., an apparatus specifically corresponding to the multi-label classification system 140 and/or the quality management system 120). The integration circuitry 216 may include hardware, software, firmware, and/or a combination thereof, designed and/or configured to request, receive, process, generate, and transmit data, data structures, control signals, and electronic information for functionality related to integration between the multi-label classification system 140 and the quality management system 120, including performing any of the operations described herein with respect to communication between the systems, accessing common data stores, generating data by one system for interpretation, parsing, and/or use by the other system, and/or causing performance of quality management operations by the quality management system 120.

In some embodiments, two or more of the sets of circuitries 202-216 are combinable. Alternatively, or additionally, one or more of the sets of circuitry 202-216 perform some or all of the operations and/or functionality described herein as being associated with another circuitry. In some embodiments, two or more of the sets of circuitry 202-216 are combined into a single module embodied in hardware, software, firmware, and/or a combination thereof. For example, in some embodiments, one or more of the sets of circuitry, for example the transformation circuitry 210, may be combined with the processor 202, such that the processor 202 performs one or more of the operations described herein with respect to the transformation circuitry 210.

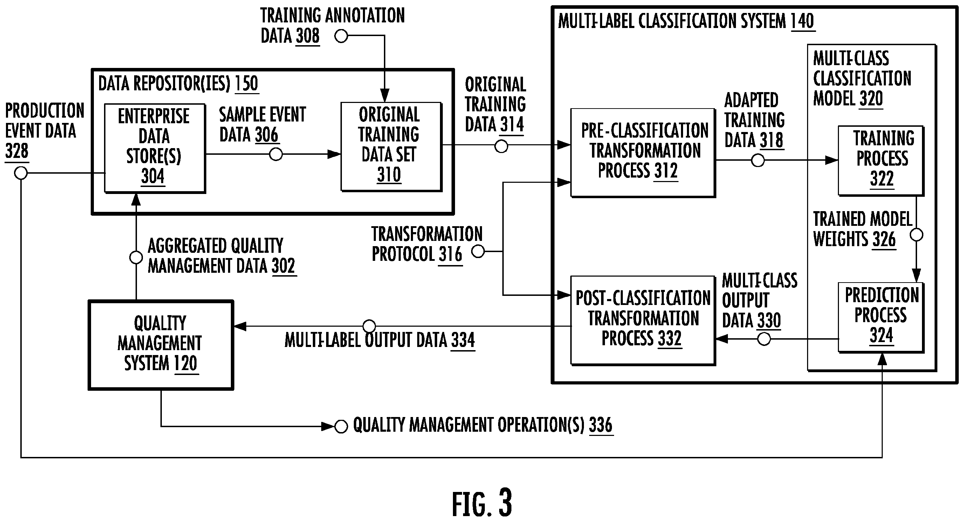

FIG. 3 is an illustration of an example multi-label classification system 140, in accordance with at least some example embodiments of the present disclosure. Specifically, FIG. 3 includes schematic depictions of the example multi-label classification system 140, including example internal processes and components of the multi-label classification system 140, and/or schematic depictions of the one or more data repositories 150 and the quality management system 120 in communication with the multi-label classification system 140, along with example data objects used by and/or produced by the depicted processes, components, and/or systems. The multi-label classification system 140 and/or apparatuses 200 associated therewith, for example, may be specially configured via hardware, software, firmware, and/or a combination thereof, to perform the various data processing and interactions described with respect to FIG. 3 to perform multi-label classification using an adapted multi-class classification model and adapted training data set with respect to one or more products produced by one or more enterprises.

In the illustrated example, the multi-label classification system 140 comprises, in some examples, a pre-classification transformation process 312, a post-classification transformation process 332, a training process 322 corresponding to a multi-class classification model 320, and a prediction process 324 corresponding to the multi-class classification model 320. Additionally, the one or more data repositories 150 comprise, in some examples, one or more enterprise data stores 304 and/or an original training data set 310.

In an example scenario, enterprise data for one or more enterprises may be generated, collected, captured, received, accumulated, and/or stored in connection with one or more products produced by the enterprise(s), one or more processes associated with production of the product(s), and/or one or more operational systems associated with the process(es) and/or product(s). Such enterprise data may be stored in one or more enterprise data stores 304 associated with the enterprise(s).

In various embodiments, the enterprise data may comprise any data associated with one or more products produced by one or more enterprises, including product data, process data, system data, operational data, any data that may be used in connection with quality management operations including quality management data and/or any data that may be included in and/or used to generate a training data set for a multi-label classification model, and/or any relevant object data and/or metadata, to list a few examples.

In one example, product data of the enterprise data stored in the enterprise data store(s) 304 may comprise various items of data and/or metadata identifying and/or characterizing the product(s) produced by the enterprise with which the enterprise data is associated, including measured, calculated, sensed, and/or otherwise determined values corresponding to various attributes associated with the product(s) in general and/or particular instances and/or batches of the product(s) that have been produced, to list a few examples.

In another example, process data of the enterprise data stored in the enterprise data store(s) may comprise various items of data and/or metadata identifying, defining, and/or characterizing the process(es) associated with the production of the product(s) (e.g., research processes, manufacturing processes, testing processes, distribution processes, quality management processes, business processes), including measured, calculated, sensed, manually configured, and/or otherwise determined values corresponding to various attributes associated with the process(es), to list a few examples.

In another example, system data of the enterprise data stored in the enterprise data store(s) may comprise various items of data and/or metadata identifying, defining, establishing configuration parameters for, and/or characterizing operational system(s) associated with the process(es) and/or products (e.g., any computing, mechanical, automation, monitoring, control, laboratory, manufacturing, and/or distribution systems or equipment), including measured, calculated, sensed, manually configured, and/or otherwise determined values corresponding to various attributes associated with the operational system(s), environments where the system(s) are installed, components (e.g., equipment, assets) comprised by the system(s), configuration parameters defining functionality of the system(s) and/or its components, and/or operational sessions during which the system(s) have previously operated, to list a few examples.

In another example, object data and/or metadata of the enterprise data stored in the enterprise data store(s) 304 may comprise various items of data and/or metadata identifying, defining, and/or characterizing various real or virtual objects associated with the product(s) or production thereof and/or any relationships between said objects.

In another example, in some embodiments, aggregated quality management data 302 of the enterprise data stored in the enterprise data store(s) 304 may comprise data received, generated, and/or aggregated by and/or within the quality management system 120, including any data associated with production of products required or utilized for quality management purposes. In some embodiments, the aggregated quality management data 302 may comprise quality management events (e.g., generated and/or logged by the quality management system based on input from various sources, including possibly internal enterprise data associated with production of the relevant products, data generated and/or received in connection with provision or use of the relevant products, and/or data characterizing and/or derived from communications with various entities associated with production, consumption, and/or use of the relevant products). In one example, the quality management events of the aggregated quality management data 302 may comprise text objects with text characterizing how products were provided, consumed, and/or used, text characterizing particular contexts in which products were provided, consumed, and/or used, text characterizing certain effects resulting from provision, consumption, and/or use of the products, among other examples, and/or text representing, characterizing, and/or describing customer complaints arising from provision, consumption, and/or use of the products subject to quality management by the quality management system 120.

Although the illustrated example concerns text objects and particularly text objects containing text from quality management events from aggregated quality management data 302 aggregated by and/or within the quality management system 120, it will be appreciated that the present disclosure may be applicable to any type of data upon which a multi-label classification is possible.

In some embodiments, the enterprise data stored in the enterprise data store(s) 304, including the aggregated quality management data 302, may comprise and/or may be generated based at least in part on input received from one or more users (e.g., via user interfaces presented on the user device(s) 160) in connection with the product(s) and/or process(es) related to production of the product(s). In one example, the aggregated quality management data 302 may comprise data characterizing and/or derived from communications with various entities (e.g., customers) associated with production, consumption, and/or use of the relevant products, including data generated based on input (e.g., user input) received via various types of forms associated with quality management operations and/or presented via the quality management system 120, including complaint forms, supplier quality management forms, audit forms, risk forms, and/or quality event forms, to list a few examples.

Moreover, in various embodiments, the enterprise data stored in the enterprise data store(s) 304 may be associated with and/or generated during or in connection with any phase of production of the product(s) and/or any corresponding process(es), including any research, manufacturing, testing, distribution, quality management, and/or business phases or processes.

In various embodiments, any of the enterprise data stored in the enterprise data store(s) 304 (including the aggregated quality management data 302) may be included in and/or used to derive an original training data set 310 corresponding to a multi-label classification task. The original training data set 310 may comprise a multi-label classification of training objects (e.g., data objects corresponding to and/or derived from the enterprise data stored in the enterprise data store(s)) each into at least one original classification set of a plurality of original classification sets. In one example, at least one of the training objects included in the original training data set 310 may be classified into a plurality of different original classification sets. In some embodiments, the multi-label classification of the training objects in the original data set 310 may be provided via training annotation data 308 included in the original training data set 310 in association with the training objects. For example, each particular training object included in the original training data set 310 may be associated with at least one classification label (e.g., provided in the training annotation data 308), each classification label corresponding to and/or representing an original classification set into which the particular training object is classified.

In some embodiments, the original training data set 310 may comprise and/or may be derived from sample event data 306 from the enterprise data store(s) 304. The sample event data 306 may comprise quality management events (e.g., from the aggregated quality management data 302), including, for example, text objects containing textual descriptions or narratives characterizing, in some examples, how products were provided, consumed, and/or used, characterizing particular contexts in which products were provided, consumed, and/or used, characterizing certain effects resulting from provision, consumption, and/or use of the products, among other examples, and/or representing, characterizing, and/or describing customer complaints arising from provision, consumption, and/or use of the products subject to quality management by the quality management system 120. The training objects of the original training data set 310 may correspond to and/or comprise the text objects from the sample event data 306. Moreover, in some embodiments, each of the original classification labels (of the training annotation data 308) may correspond to and/or represent a particular MedDRA term (as defined and described above) such that each of the training objects (e.g., text objects) may be associated with at least one MedDRA term in the original training data set 310. For example, association between a text object (of the sample event data 306) and a MedDRA term (of the training annotation data 308) may be indicative of a determined relevance of the associated MedDRA term to the content of the text object (e.g., the conditions and/or situation described within the text of the text object). In some embodiments, the training annotation data 308 may be generated based at least in part on user input (e.g., received via a user interface) with respect to the text objects in the sample event data 306, such user input, in one example, being received from a user that is a subject matter expert. In some embodiments, the training annotation data 308 may be generated automatically (e.g., via one or more analysis algorithms) and/or may be native to the sample event data 306 (e.g., corresponding to features or attributes already present in the sample event data 306). Additionally or alternatively, the original training data set 310 may comprise data sufficient for training of a multi-label classification model to perform a multi-label classification task.

In various embodiments, the multi-label classification system 140 may be configured to perform a multi-label classification task with respect to a given input data set by using an adapted multi-class classification model 320.

More particularly, the multi-label classification system 140 may be configured to perform the multi-label classification task using the adapted multi-class classification model 320 in conjunction with pre-classification processing functionality for adapting multi-label training data (e.g., the original training data set 310) for use with the multi-class classification model 320 by transforming the multi-label training data into multi-class training data. For example, in some embodiments, the pre-classification transformation process 312 of the multi-label classification system 140 may be configured to receive, retrieve, and/or access original training data 314 (from the original training data set 310 stored in the one or more data repositories 150). The original training data 314 may correspond to the data comprised by the original training data set 310 and/or may comprise some or all of the data of the original training data set 310 as defined and described above. The pre-classification transformation process 312 may be configured to transform the original training data 314 into adapted training data 318, for example, based at least in part on a predefined transformation protocol 316.

In some embodiments, the transformation protocol 316 may comprise data defining particular values and/or instructions that determine the particular transformation behavior and/or functionality of the pre-classification transformation process 312 and the post-classification transformation process 332. In one example, the transformation protocol 316 may define a particular format for the adapted training data 318 expected by the multi-class classification model 320 and/or a particular format for multi-label output data 334 ultimately produced by the multi-label classification system 140.

In some embodiments, the adapted training data 318 may comprise a multi-class classification of each of the training objects of the original training data 314 into a corresponding combined classification set (e.g., from a set of at least one combined classification set). In one example, the combined classification set corresponding to each particular training object in the adapted training data 318 may represent a combination of each original classification set into which the particular training object is classified in the multi-label classification provided in the original training data 314. Put another way, a training object classified into multiple individual classification sets in the original training data 314 may be transformed such that, in the adapted training data 318, that training object is classified into a single combined classification set representing all of the multiple individual classification sets in aggregate.

In one example, transforming (e.g., by the pre-classification transformation process 312) the original training data 314 into the adapted training data 318 may comprise, for each particular training object of those classified in the original training data 314, combining all original classification labels corresponding to original classification sets into which the particular training object is classified in the original training data 314 into a combined classification label comprising all of the original classification labels for the particular training object. In some embodiments, the transformation protocol 316 may define a label combiner operator (e.g., by designating a particular character as the label combiner operator), which label combiner operator may represent a delimiter between individual original classification labels in the combined classification label. The pre-classification transformation process 312 may be configured to generate a combined classification label for a particular training object to include each individual original classification label associated with the particular training object with an instance of the label combiner operator interposed between each pair of individual original classification labels. In various embodiments, the pre-classification transformation process 312 may be configured to generate a combined classification label to be interpretable (e.g., by the post-classification transformation process 332) in order to determine from any given combined classification label the one or more individual original classification labels that make up that combined classification label. In some embodiments, the pre-classification transformation process 312 may be configured to generate a mapping of each combined classification label reflected in the adapted training data 318 to a corresponding one or more individual original classification labels.

In various embodiments, the multi-class classification model 320 may be a machine learning model for implementing a multi-class classification with respect to a given input data set, in which each object of the input data set is classified into exactly one classification set from among a plurality of classification sets including at least three classification sets. In one example, a particular instance of the multi-class classification model 320 may be trained for a particular classification task (e.g., classifying certain types of objects into certain classification sets based on certain features associated with the objects being classified) using a training data set, and such a particular instance of the multi-class classification model 320, as trained using the training data set, may be used, in turn, to generate multi-class classifications with respect to a given input data set. The multi-label classification system 140 may be configured to train the multi-class classification model 320 based at least in part on the adapted training data 318 and to generate multi-class output data 330 with respect to an input data set using the trained multi-class classification model 320.

More particularly, the multi-class classification model 320 may be configured by and/or based at least in part on the training process 322 and the prediction process 324.

In various embodiments, the multi-class classification model 320 may undergo a training operation (e.g., performed by the training process 322) using a training data set. The training data set may comprise the adapted training data 318 (as defined and described above) along with possibly any data associated with training objects included in the adapted training data 318 that is relevant to the particular prediction task that the multi-class classification model 320 is being trained to perform, including any portions of the enterprise data and/or aggregated quality management data 302.

In various embodiments, an instance of the multi-class classification model 320 may comprise a data object created by using machine learning to learn to perform a given function (e.g., a prediction) through training with the training data set (e.g., adapted training data 318. The multi-class classification model 320 may be trained to generate a predictions (e.g., included in the multi-class output data 330) by learning from the training data set. For example, the multi-class classification model 320 may comprise a mapping function *f* from input variables *x* to discrete output variables y. The training process 322 may be configured to formulate a particular mapping function *f* from input variables *x* (corresponding to features of the training data set and an input data set associated with a particular object) to a discrete output variable y (representing a predicted classification set into which the particular object should be classified).

More particularly, in one example, the multi-class classification model 320 may undergo training (e.g., by the training process 322) using the training data set (e.g., including the adapted training data 318) in order to identify features from the training data set and to determine optimal coefficients representing adjustment or weights to apply with respect to the features in order to produce a target value reflected in the training data set, for example, based on positive and/or negative correlations between the target values and various features of the training data set. The target value for the multi-class classification model 320 may represent a classification of an object into a predicted classification set and may correspond to the combined classification sets (e.g., as represented by the combined classification labels) indicated for each training object in the adapted training data 318. The features may correspond to various domain-specific attributes reflected in the training data set, including characteristics of the training objects (e.g., text objects) included in the adapted training data 318 and/or any attributes associated with the training objects from the sample event data 306, aggregated quality management data 302, and/or any other enterprise data stored in the enterprise data store(s) 304.

In some embodiments, the training data set (e.g., comprising at least in the adapted training data 318) may be input into the training process 320 corresponding to the multi-class classification model 320 to train the model(s) to generate the predictions (e.g., defined in the multi-class output data 330). In one example, for the multi-class classification model 320, a product of the model training (e.g., by the training process 322) are trained model weights 326 that are used by the prediction process 324 corresponding to the multi-class classification model 320. In some embodiments, after an initial training, further training data (e.g., including subsequently received and/or generated sample event data 306) may be input to the training process 322 corresponding to the multi-class classification model 320, periodically or on an on-going basis, to refine and update the model(s).

The multi-class classification model 320 may be trained (e.g., via the training process 322) to generate a prediction (e.g., included in the multi-class output data 330) based at least in part on an input data set, which may be embodied in and/or comprise production event data 328, for example.

In some embodiments, an input data set (e.g., embodied in and/or comprising the production event data 328) for generating a prediction (e.g., included in the multi-class output data 330) may comprise a current instance (e.g., reflecting current or up-to-date data at an instance of time when the prediction is being generated) of any of the enterprise data and/or aggregated quality management data 302 stored in the enterprise data store(s) 304, and/or any other data relevant to the multi-class classification task being performed. In one example, the input data set (e.g., embodied in and/or comprising the production event data 328) may comprise production objects (e.g., text objects) of the same type as and/or analogous to the training objects reflected in the training data set (e.g., including the adapted training data 318), including any associated attributes or features, but without any classification of the production objects into classification sets, and the prediction process 324 may be configured to generate the multi-class output data 330 to comprise a classification of the production objects into classification sets based on the training of the model performed by the training process 322 as defined and described above. In some embodiments, the production event data 328 may comprise quality management events (e.g., from the aggregated quality management data 302), including, for example, text objects containing textual descriptions or narratives characterizing, in some examples, how products were provided, consumed, and/or used, characterizing particular contexts in which products were provided, consumed, and/or used, characterizing certain effects resulting from provision, consumption, and/or use of the products, among other examples, and/or representing, characterizing, and/or describing customer complaints arising from provision, consumption, and/or use of the products subject to quality management by the quality management system 120. The production objects of the input data set may correspond to and/or comprise the text objects from the production event data 328.

In some embodiments, an input data set (e.g., embodied in and/or comprising the production event data 328) may be input into the prediction process 324 corresponding to the multi-class classification model 320, and, in response to receiving the input data set, the prediction process 324 corresponding to the multi-class classification model 320 may be configured to generate the multi-class output data 330 with respect to the input data set based at least in part on the input data set and/or on the trained model weights 326 generated during training of the model.

The multi-class output data 330 may comprise a multi-class classification of production objects from the input data set each into exactly one classification set from among a plurality of classification sets (namely, the set of combined classification sets reflected in the adapted training data 318 used to train the model). In one example, the multi-class output data 330 may comprise a classification of the production objects from the input data set each into a corresponding combined classification set of the at least one combined classification set from the adapted training data 318.

In various embodiments, the multi-label classification system 140 may be configured to perform the multi-label classification task using the adapted multi-class classification model 320 in conjunction with post-classification processing functionality for transforming a multi-class classification into a multi-label classification suitable for use in a present enterprise management context (e.g., performing quality management operations). For example, in some embodiments, the post-classification transformation process 332 of the multi-label classification system 140 may be configured to receive the multi-class output data 330 output by the prediction process 324 corresponding to the multi-class classification model 320. The post-classification transformation process 332 may be configured to transform the multi-class output data 330 into multi-label output data 334, for example, based at least in part on the transformation protocol 316.

In some embodiments, the multi-label output data 334 may comprise a multi-label classification of production objects from the input data set each into at least one classification set from among a plurality of classification sets. In one example, the multi-label output data 334 may comprise a classification of the production objects from the input data set each into at least one original classification set of the plurality of original classification sets from the original training data set 310 and/or original training data 314. In some scenarios, at least one production object of those classified in the multi-label output data 334 may be classified into a plurality of different original classification sets.

In one example, transforming (e.g., by the post-classification transformation process 332) the multi-class output data 330 into the multi-label output data 334 may comprise, for each particular production object of the production objects classified in the multi-class output data 330, parsing a combined classification label corresponding to a combined classification set into which the particular production object is classified in the multi-class output data 330 into at least one original classification label, corresponding to an original classification set from the original training data set 310 and/or original training data 314, into which the particular production object is to be classified in the multi-label output data 334. Put another way, the post-classification transformation process 332 may be configured to parse the combined classification label for each particular production object in order to determine which original classification set(s) into which the particular production object should be classified and/or which original classification label(s) to assign to the particular production object. In some embodiments, the post-classification transformation process 332 may be configured to parse the combined classification labels based at least in part on the transformation protocol 316, including, for example, the label combiner operator defined by the transformation protocol 316. For example, the post-classification transformation process 332 may be configured extract from each combined classification label a series of one or more individual original classification labels contained within the combined classification label based on any instances of the label combiner operator found in the combined classification label and the delimitation of the individual original classification labels within the combined classification label indicated by the placement of the instances of the label combiner operator with respect to the various individual original classification labels. Moreover, in some embodiments, the transformation protocol 316 may define a mapping (e.g., generated by the pre-classification transformation process 312 in the course of generating the adapted training data 318) of each combined classification label reflected in the adapted training data 318 to a corresponding one or more individual original classification labels from the original data set 310 and/or the original training data 314. The post-classification transformation process 332 may be configured to determine the individual original classification label(s) to assign to each production object based at least in part on the mapping of combined classification labels to original classification labels defined in the transformation protocol 316.

In various embodiments, the multi-label classification system 140 may be configured to cause performance (e.g., by the quality management system 120) of at least one enterprise management operation based at least in part on the multi-label output data 334. In one example, the enterprise management operation(s) caused to be performed by the multi-label classification system 140 based on the multi-label output data 334 may include one or more quality management operation(s) 336. For example, in some example scenarios and/or in some embodiments, the quality management system 120 may be configured to receive the multi-label output data 334 from the multi-label classification system 140 to perform the quality management operation(s) 336 (e.g., with respect to the various research, development, manufacturing, distribution, and/or service provision processes associated with production of the product(s) subject to quality management by the quality management system 120). In one example, based at least in part on the multi-label output data 334, the quality management system 120 may be configured to perform quality management operations 336 including updating a GUI of the quality management system 120 (e.g., to display the multi-label output data 334 and/or data derived therefrom in a particular manner), generating and/or transmitting alerts, generating, organizing, and/or formatting reports, and/or performing automated operations (e.g., generating and submitting orders, scheduling service and/or inspection, generating related quality management events, performing automatic service and/or diagnostic operations), to list a few examples.

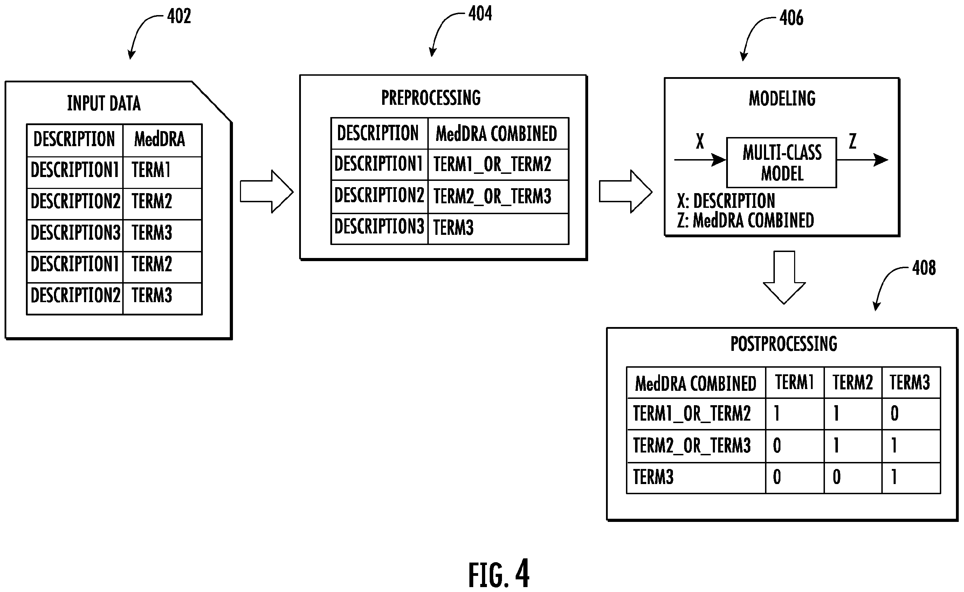

FIG. 4 is an illustration of example data objects used by and/or produced by the multi-label classification system 140 at various stages of adapting a multi-class classification model for use in performing a multi-label classification.

At a first stage 402, an example original training data set (e.g., corresponding to and/or comprising the original training data set 310 and/or the original training data 314) is shown in its form before transformation by the multi-label classification system 140 into an adapted training data set. In particular, the illustrated example concerns classification of text objects (e.g., from quality management events) into one or more classification sets corresponding to MedDRA terms as defined and described above. The original training data set at the first stage 402 specifies values corresponding to a "Description" attribute and a "MedDRA" attribute with respect to a series of training objects (e.g., text objects). The "Description" attribute may identify a particular training object (e.g., a particular text object containing a textual description of a situation or conditions related to provision of a product produced by an enterprise) while the associated "MedDRA" attribute may indicate an original classification label associated with the training object identified via the "Description" attribute. Notably, in the example original training data set shown at the first stage 402, training objects may be associated with multiple original classification sets, for example, with the various training objects having a "many to many" correspondence with the various classification labels. In this way, for example, the original training data set shown with respect to the first stage 402 may correspond to a multi-label classification task. In the illustrated example, the training object identified as "Description1" is classified into both a classification set represented by a "Term1" classification label (e.g., corresponding to a particular MedDRA term) and a classification set represented by a "Term2" classification label, the training object identified as "Description2" is classified into both the classification set represented by the "Term2" classification label and a classification set represented by a "Term3" classification label, and the training object identified as "Descriptions" is classified into only the classification set represented by the "Term3" classification label.

At a second stage 404, an example adapted training data set (e.g., corresponding to and/or comprising the adapted training data 318) is shown in its form after transformation by the multi-label classification system 140 from the original training data set shown with respect to the first stage 402. As with the original training data set at the first stage 402, the adapted training data set shown at the second stage 404 specifies values corresponding to the "Description" attribute with respect to a series of training objects, the specified values identifying particular training objects (from among those indicated in the original training data set shown with respect to the first stage 402). Now, however, each of the training objects is assigned a value corresponding to a "MedDRA combined" attribute, which may represent a combined classification label (as defined and described with respect to FIG. 3, for example). Notably, in the example original training data set shown at the second stage 404, training objects may be associated with exactly one combined classification set, for example, with the various training objects having a "one to one" correspondence with the various classification labels. In this way, for example, the adapted training data set shown with respect to the first stage 402 may correspond to a multi-class classification task. Each of the "MedDRA combined" attributes corresponding to combined classification labels may represent a combination of one or more of the "MedDRA" attributes provided in the original training data set shown with respect to the first stage 402. For "MedDRA combined" attributes representing a combination of multiple "MedDRA" attributes, the original "MedDRA" classification labels are both included in the corresponding "MedDRA combined" attributes delimited via a label combiner operator "_or_" (e.g., as defined in the transformation protocol) interposed between the individual original "MedDRA" labels.

In the illustrated example, at the second stage 404, the training object identified as "Description1" is classified into a single combined classification set represented by a "Term1_or_Term2" combined classification label by virtue of the corresponding training object being labeled as both "Term1" and "Term2" in the original training data set. Similarly, the training object identified as "Description2" is classified into a single combined classification set represented by a "Term2_or_Term3" combined classification label by virtue of the corresponding training object being labeled as both "Term2" and "Term3" in the original training data set. The the training object identified as "Descriptions" is classified into a single combined classification set represented by a "Term3" combined classification label, which is unchanged from the "Term3" original classification label by virtue of the corresponding training object being labeled as only "Term3" in the original training data set.

At a third stage 406, an example multi-class classification model (e.g., corresponding to and/or comprising the multi-class classification model 320) is shown. The example multi-class classification model is configured to, for each input variable X corresponding to a (training or production) object (e.g., a description analogous to those identified in the original training data set shown with respect to the first stage 402 and those identified in the adapted training data set shown with respect to the second stage 404), generate a corresponding output variable Z corresponding to a predicted "MedDRA combined" combined classification label from the adapted training data set shown with respect to the second stage 404.

At a fourth stage 408, an example mapping of combined classification labels reflected in the adapted training data to corresponding individual original classification label(s), which mapping may be used to transform multi-class output data produced by the model shown with respect to the third stage 406 into multi-label output data. In various embodiments, this mapping may be generated based on the set of combined classification labels reflected in the adapted training data set, for example, in conjunction with transformation of the original training data set into the adapted training data set or in conjunction with transforming the multi-class output data into the multi-label output data. In on example, the mapping may be generated based at least in part on the transformation protocol, particularly based at least in part on the label combiner operator defined in the transformation protocol, for example, by parsing the "MedDRA combined" combined classification labels with respect to the label combiner operator in order to determine which "MedDRA" original classification labels correspond with each "MedDRA combined" combined classification label. In the mapping, each "MedDRA combined" combined classification label is associated with a binary value corresponding to each particular "MedDRA" original classification label from the original training data set ("Term1", "Term2", "Term3"), with a first binary value "1" indicative of a corresponding "MedDRA" original classification label being mapped to the associated "MedDRA combined" combined classification label and a second binary value "0" indicative of a corresponding "MedDRA" original classification label not being mapped to the associated "MedDRA combined" combined classification label.

In the illustrated example, the "Term1_or_Term2" combined classification label is mapped to the "Term1" original classification label and to the "Term2" original classification label but not to the "Term3" original classification label. Accordingly, any objects (e.g., production objects from an input data set) assigned the "Term1_or_Term2" combined classification label in the multi-class output data will be assigned both the "Term1" original classification label and the "Term2" original classification label in the corresponding multi-label output data (that is generated by transforming the multi-class output data). Similarly, the "Term2_or_Term3" combined classification label is mapped to the "Term2" original classification label and to the "Term3" original classification label but not to the "Term1" original classification label. Accordingly, any objects assigned the "Term2_or_Term3" combined classification label in the multi-class output data will be assigned both the "Term2" original classification label and the "Term3" original classification label in the corresponding multi-label output data. Finally, the "Term3" combined classification label is mapped only to the "Term3" original classification label. Accordingly, any objects assigned the "Term3" combined classification label in the multi-class output data will be assigned only the "Term3" original classification label in the corresponding multi-label output data.

Having described example systems and/or apparatuses of the present disclosure, example flowcharts including various operations performed by the apparatuses and/or systems described herein will now be discussed. It should be appreciated that each of the flowcharts depicts an example computer-implemented process that may be performed by one or more of the apparatuses, systems, and/or devices described herein, for example utilizing one or more of the components thereof. The blocks indicating operations of each process may be arranged in any of a number of ways, as depicted and described herein. In some such embodiments, one or more blocks of any of the processes described herein occur in-between one or more blocks of another process, before one or more blocks of another process, and/or otherwise operates as a sub-process of a second process. Additionally or alternatively, any of the processes may include some or all of the steps described and/or depicted, including one or more optional operational blocks in some embodiments. With respect to the flowcharts discussed below, one or more of the depicted blocks may be optional in some, or all, embodiments of the disclosure. Similarly, it should be appreciated that one or more of the operations of each flowchart may be combinable, replaceable, and/or otherwise altered as described herein.

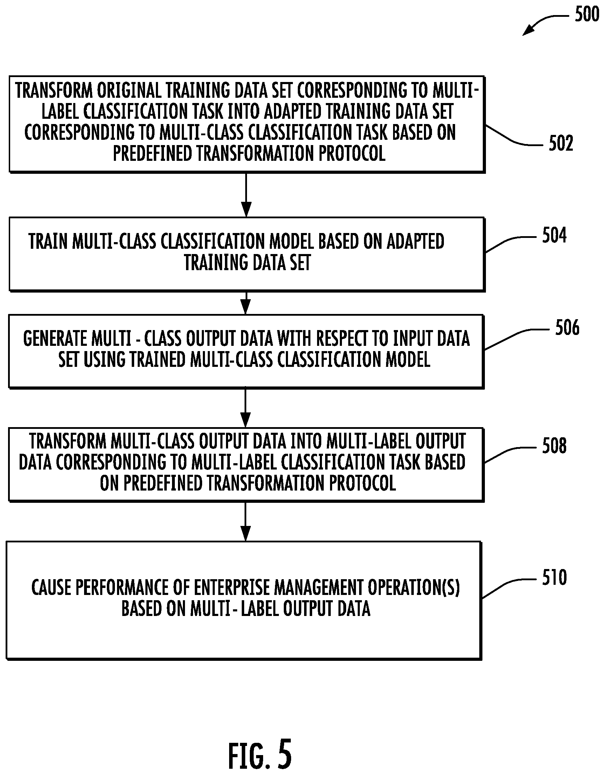

FIG. 5 illustrates a flowchart including operational blocks of an example process in accordance with at least some example embodiments of the present disclosure. In some embodiments, the computer-implemented process of FIG. 5 is embodied by computer program code stored on a non-transitory computer-readable medium of a computer program product configured for execution to perform the computer-implemented method. Alternatively or additionally, in some embodiments, the example process of FIG. 5 is performed by one or more specially configured computing devices, such as the specially configured apparatus 200 (e.g., via transformation circuitry 210, training circuitry 212, classification circuitry 214, and/or integration circuitry 216). In this regard, in some such embodiments, the apparatus 200 is specially configured by computer program instructions stored thereon, for example in the memory 204 and/or another component depicted and/or described herein, and/or otherwise accessible to the apparatus 200, for performing the operations as depicted and described with respect to the example process of FIG. 5. In some embodiments, the specially configured apparatus 200 includes and/or otherwise is in communication with one or more external apparatuses, systems, devices, and/or the like, to perform one or more of the operations as depicted and described. While the operational blocks of the example process are depicted in FIG. 5 in a particular order, this should not be understood as requiring that such operations be performed in the particular order shown or in sequential order, or that all illustrated operations be performed.

FIG. 5 illustrates a flowchart including operational blocks of an example process 500 for performing multi-label classification using an adapted multi-class classification model, in accordance with at least some example embodiments of the present disclosure.

The process 500 begins at operation 502, at which an apparatus (such as, but not limited to, the apparatus 200 or circuitry thereof as described above in connection with FIG. 2) transforms an original training data set corresponding to a multi-label classification task into an adapted training data set corresponding to a multi-class classification task based at least in part on a predefined transformation protocol. In various embodiments, the original training data set transformed at operation 502 may correspond to and/or comprise the original training data set 310 and/or the original training data 314 as defined and described with respect to FIG. 3, the adapted training data set generated at operation 502 may correspond to and/or comprise the adapted training data 318 and/or any training data set comprising the adapted training data 318 as defined and described with respect to FIG. 3, and transforming the original training data set into the adapted training data set at operation 502 may comprise some or all of the analogous functionality attributed to the pre-classification transformation process 312 as described with respect to FIG. 3. Additionally or alternatively, the predefined transformation protocol referenced with respect to operation 502 may correspond to and/or comprise the transformation protocol 316 as defined and described with respect to FIG. 3.

At operation 504 of the process 500, an apparatus (such as, but not limited to, the apparatus 200 or circuitry thereof described above in connection with FIG. 2) trains a multi-class classification model based at least in part on the adapted training data set generated at operation 502. In various embodiments, the multi-class classification model trained at operation 504 may correspond to and/or comprise the multi-class classification model 320 as defined and described with respect to FIG. 3, and training the multi-class classification model at operation 504 may comprise some or all of the analogous functionality attributed to the training process 322 as described with respect to FIG. 3.

At operation 506 of the process 500, an apparatus (such as, but not limited to, the apparatus 200 or circuitry thereof described above in connection with FIG. 2) generates multi-class output data with respect to an input data set using the multi-class classification model trained at operation 504. In various embodiments, the input data set referenced with respect to operation 506 may correspond to and/or comprise the production event data 328 and/or any input data set comprising the production event data 328 as defined and described with respect to FIG. 3, the multi-class output data generated at operation 506 may correspond to and/or comprise the multi-class output data 330 as defined and described with respect to FIG. 3, and generating the multi-class output data 330 at operation 506 may comprise some or all of the analogous functionality attributed to the prediction process 324 as described with respect to FIG. 3.

At operation 508 of the process 500, an apparatus (such as, but not limited to, the apparatus 200 or circuitry thereof described above in connection with FIG. 2) transforms the multi-class output data generated at operation 506 into multi-label output data corresponding to the multi-label classification task (referenced with respect to operation 502) based at least in part on the predefined transformation protocol (referenced with respect to operation 502). In various embodiments, the multi-label output data generated at operation 508 may correspond to and/or comprise the multi-label output data 334 as defined and described with respect to FIG. 3, and generating the multi-label output data 334 at operation 508 may comprise some or all of the analogous functionality attributed to the post-classification transformation process 332 as described with respect to FIG. 3.

At operation 510 of the process 500, an apparatus (such as, but not limited to, the apparatus 200 or circuitry thereof described above in connection with FIG. 2) causes performance of at least one enterprise management operations based at least in part on the multi-label output data generated at operation 508. In various embodiments, the enterprise management operation(s) caused to be performed at operation 510 may correspond to and/or comprise the quality management operation(s) 336 as defined and described with respect to FIG. 3.

Although example processing systems have been described in the figures herein, implementations of the subject matter and the functional operations described herein can be implemented in other types of digital electronic circuitry, or in computer software, firmware, or hardware, including the structures disclosed in this specification and their structural equivalents, or in combinations of one or more of them.

Embodiments of the subject matter and the operations described herein can be implemented in digital electronic circuitry, or in computer software, firmware, or hardware, including the structures disclosed in this specification and their structural equivalents, or in combinations of one or more of them. Embodiments of the subject matter described herein can be implemented as one or more computer programs, i.e., one or more modules of computer program instructions, encoded on computer storage medium for execution by, or to control the operation of, information/data processing apparatus. Alternatively, or in addition, the program instructions can be encoded on an artificially-generated propagated signal, e.g., a machine-generated electrical, optical, or electromagnetic signal, which is generated to encode information/data for transmission to suitable receiver apparatus for execution by an information/data processing apparatus. A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially-generated propagated signal. The computer storage medium can also be, or be included in, one or more separate physical components or media (e.g., multiple CDs, disks, or other storage devices).

The operations described herein can be implemented as operations performed by an information/data processing apparatus on information/data stored on one or more computer-readable storage devices or received from other sources.

The term "data processing apparatus" encompasses all kinds of apparatus, devices, and machines for processing data, including by way of example a programmable processor, a computer, a system on a chip, or multiple ones, or combinations, of the foregoing. The apparatus can include special purpose logic circuitry, e.g., an FPGA (field programmable gate array) or an ASIC (application-specific integrated circuit). The apparatus can also include, in addition to hardware, code that creates an execution environment for the computer program in question, e.g., code that constitutes processor firmware, a protocol stack, a repository management system, an operating system, a cross-platform runtime environment, a virtual machine, or a combination of one or more of them. The apparatus and execution environment can realize various different computing model infrastructures, such as web services, distributed computing and grid computing infrastructures.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or information/data (e.g., one or more scripts stored in a markup language document), in a single file dedicated to the program in question, or in multiple coordinated files (e.g., files that store one or more modules, sub-programs, or portions of code). A computer program can be deployed to be executed on one computer or on multiple computers that are located at one site or distributed across multiple sites and interconnected by a communications network.

The processes and logic flows described herein can be performed by one or more programmable processors executing one or more computer programs to perform actions by operating on input information/data and generating output. Processors suitable for the execution of a computer program include, by way of example, both general and special purpose microprocessors, and any one or more processors of any kind of digital computer. Generally, a processor will receive instructions and information/data from a read-only memory or a random access memory or both. The essential elements of a computer are a processor for performing actions in accordance with instructions and one or more memory devices for storing instructions and data. Generally, a computer will also include, or be operatively coupled to receive information/data from or transfer information/data to, or both, one or more mass storage devices for storing data, e.g., magnetic, magneto-optical disks, or optical disks. However, a computer need not have such devices. Devices suitable for storing computer program instructions and information/data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry.

To provide for interaction with a user, embodiments of the subject matter described herein can be implemented on a computer having a display device, e.g., a CRT (cathode ray tube) or LCD (liquid crystal display) monitor, for displaying information/data to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

Embodiments of the subject matter described herein can be implemented in a computing system that includes a back-end component, e.g., as an information/data server, or that includes a middleware component, e.g., an application server, or that includes a front-end component, e.g., a client computer having a graphical user interface or a web browser through which a user can interact with an implementation of the subject matter described herein, or any combination of one or more such back-end, middleware, or front-end components. The components of the system can be interconnected by any form or medium of digital information/data communication, e.g., a communications network. Examples of communications networks include a local area network ("LAN") and a wide area network ("WAN"), an inter-network (e.g., the Internet), and peer-to-peer networks (e.g., ad hoc peer-to-peer networks).

The computing system can include clients and servers. A client and server are generally remote from each other and typically interact through a communications network. The relationship of client and server arises by virtue of computer programs running on the respective computers and having a client-server relationship to each other. In some embodiments, a server transmits information/data (e.g., an HTML page) to a client device (e.g., for purposes of displaying information/data to and receiving user input from a user interacting with the client device). Information/data generated at the client device (e.g., a result of the user interaction) can be received from the client device at the server.

While this specification contains many specific implementation details, these should not be construed as limitations on the scope of any disclosures or of what may be claimed, but rather as descriptions of features specific to particular embodiments of particular disclosures. Certain features that are described herein in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

Thus, particular embodiments of the subject matter have been described. Other embodiments are within the scope of the following claims. In some cases, the actions recited in the claims can be performed in a different order and still achieve desirable results. In addition, the processes depicted in the accompanying figures do not necessarily require the particular order shown, or sequential order, to achieve desirable results. In certain implementations, multitasking and parallel processing may be advantageous.

It is to be understood that the disclosure is not to be limited to the specific embodiments disclosed, and that modifications and other embodiments are intended to be included within the scope of the appended claims. Although specific terms are employed herein, they are used in a generic and descriptive sense only and not for purposes of limitation, unless described otherwise.

## Claims

1. An apparatus comprising at least one processor and at least one non-transitory memory comprising program code stored thereon, wherein the at least one non-transitory memory and the program code are configured to, with the at least one processor, cause the apparatus to at least:
transform an original training data set corresponding to a multi-label classification task into an adapted training data set corresponding to a multi-class classification task based at least in part on a predefined transformation protocol, wherein a multi-class classification model configured to perform the multi-class classification task is trained based at least in part on the adapted training data set;
transform multi-class output data generated with respect to an input data set using the trained multi-class classification model into multi-label output data corresponding to the multi-label classification task based at least in part on the predefined transformation protocol; and
cause performance of at least one enterprise management operation based at least in part on the multi-label output data.

2. The apparatus of claim 1, wherein the original training data set comprises a multi-label classification of training objects each into at least one original classification set of a plurality of original classification sets, in which at least one training object of the training objects is classified into a plurality of different original classification sets.

3. The apparatus of claim 2, wherein the adapted training data set comprises a multi-class classification of the training objects each into a corresponding combined classification set of at least one combined classification set, in which the combined classification set corresponding to each particular training object of the training objects represents a combination of each original classification set into which the particular training object is classified in the multi-label classification of the original training data set.

4. The apparatus of claim 3, wherein the multi-class output data comprises a classification of production objects from the input data set each into a corresponding combined classification set of the at least one combined classification set from the adapted training data set.

5. The apparatus of claim 4, wherein the multi-label output data comprises a classification of the production objects from the input data set each into at least one original classification set of the plurality of original classification sets from the original training data set.

6. The apparatus of claim 1, wherein the at least one non-transitory memory and the program code are configured to, with the at least one processor, further cause the apparatus to at least:
train the multi-class classification model based at least in part on the adapted training data set.

7. The apparatus of claim 1, wherein the at least one non-transitory memory and the program code are configured to, with the at least one processor, further cause the apparatus to at least:
generate the multi-class output data with respect to the input data set using the trained multi-class classification model.

8. The apparatus of claim 1, wherein transforming the original training data set into the adapted training data set comprises, for each particular training object of training objects classified in the original training data set, combining all original classification labels corresponding to original classification sets into which the particular training object is classified in the original training data set into a combined classification label comprising all of the original classification labels for the particular training object delimited via a label combiner operator defined according to the predefined transformation protocol.

9. The apparatus of claim 1, wherein transforming the multi-class output data into the multi-label output data comprises, for each particular production object of production objects classified in the multi-class output data, parsing, based at least in part on a label combiner operator defined according to the predefined transformation protocol, a combined classification label corresponding to a combined classification set into which the particular production object is classified in the multi-class output data into at least one original classification label corresponding to an original classification set from the original training data set into which the particular production object is to be classified in the multi-label output data, wherein the combined classification label comprises the at least one original classification label delimited via the label combiner operator.

10. The apparatus of claim 1, wherein the multi-label classification task includes classification of text objects containing text characterizing quality management events associated with products produced by an enterprise into classification sets corresponding to medical terms from a medical dictionary.

11. A computer-implemented method comprising:
transforming an original training data set corresponding to a multi-label classification task into an adapted training data set corresponding to a multi-class classification task based at least in part on a predefined transformation protocol, wherein a multi-class classification model configured to perform the multi-class classification task is trained based at least in part on the adapted training data set;
transforming multi-class output data generated with respect to an input data set using the trained multi-class classification model into multi-label output data corresponding to the multi-label classification task based at least in part on the predefined transformation protocol; and
cause performance of at least one enterprise management operation based at least in part on the multi-label output data.

12. The method of claim 11, wherein the original training data set comprises a multi-label classification of training objects each into at least one original classification set of a plurality of original classification sets, in which at least one training object of the training objects is classified into a plurality of different original classification sets.

13. The method of claim 12, wherein the adapted training data set comprises a multi-class classification of the training objects each into a corresponding combined classification set of at least one combined classification set, in which the combined classification set corresponding to each particular training object of the training objects represents a combination of each original classification set into which the particular training object is classified in the multi-label classification of the original training data set.

14. The method of claim 13, wherein the multi-class output data comprises a classification of production objects from the input data set each into a corresponding combined classification set of the at least one combined classification set from the adapted training data set.

15. A computer program product comprising at least one non-transitory computer-readable storage medium having computer-readable program code portions stored therein, the computer-readable program code portions comprising an executable portion configured to:
transform an original training data set corresponding to a multi-label classification task into an adapted training data set corresponding to a multi-class classification task based at least in part on a predefined transformation protocol, wherein a multi-class classification model configured to perform the multi-class classification task is trained based at least in part on the adapted training data set;
transform multi-class output data generated with respect to an input data set using the trained multi-class classification model into multi-label output data corresponding to the multi-label classification task based at least in part on the predefined transformation protocol; and
cause performance of at least one enterprise management operation based at least in part on the multi-label output data.
